# EUROPEAN PATENT APPLICATION

(11) **EP 4 516 393 A1**
(43) Date of publication of application: **05.03.2025**
(21) Application number: 23796372.3
(22) Date of filing: 25.04.2023
(51) Int. Cl.: B01J 19/00, B01D 3/42, C07C 1/04, C07C 1/12, C07C 9/04, C07C 29/152, C07C 31/04, C07C 41/05, C07C 43/04, C10L 3/08, G05B 23/02

(54) **PRODUCTION DEVICE**

(30) Priority: 29.04.2022 JP 2022075611
(71) Applicant: Niterra Co., Ltd., Nagoya-shi, Aichi 461-0005 (JP)
(72) Inventor: YASUDA Yutaka, Nagoya-shi, Aichi 461-0005 (JP); YABUHANA Masaki, Nagoya-shi, Aichi 461-0005 (JP)
(74) Representative: Grünecker Patent- und Rechtsanwälte PartG mbB
(86) International application number: PCT/JP2023/016262
(87) International publication number: WO 2023/210632

(57) **Abstract**

Provided is a production device, the state of which can be monitored immediately. The production device (10) is for obtaining, through chemical reaction or purification, a target product from at least one raw material of liquid or gas, and comprises: a variable acquisition unit (46) for acquiring a first variable inputted to the production device and a second variable including at least one state variable related to the target product and intermediates produced in the process of obtaining the target product from the raw material; and an estimation unit (47) for estimating the state of the production device on the basis of the first variable and the second variable acquired by the variable acquisition unit.

## Description

### TECHNICAL FIELD

The present invention relates to a production device for obtaining a target product through a chemical reaction or purification.

### BACKGROUND ART

Patent Literature 1 discloses a prior art pertaining to a production device for obtaining a target product, from at least one type of raw material selected from liquid and gaseous materials, through a chemical reaction or purification. Specifically, Patent Literature 1 discloses a prior art pertaining to a production device which manufactures fuel using hydrogen and carbon dioxide separated and recovered from exhaust gas.

### CITATION LIST

### PATENT LITERATURE

Patent Literature 1: JP2009-77457A

### SUMMARY OF INVENTION

### TECHNICAL PROBLEM

The prior art has a problem of inability to monitor the state of the production device in real time.

The present invention has been made to solve this problem, and an object of the present invention is to provide a production device that can monitor its state in real time.

### SOLUTION TO PROBLEM

In order to achieve this object, the present invention provides a production device for obtaining a target product from at least one type of raw material selected from liquid and gaseous materials through a chemical reaction or purification, the production device comprising:
a variable acquisition section which acquires a first variable inputted to the production device and second variables including at least one state variable related to the target product and an intermediate produced in a process of obtaining the target product from the raw material; and
an estimation section which estimates the state of the production device on the basis of the first variable and the second variables acquired by the variable acquisition section.

### ADVANTAGEOUS EFFECTS OF INVENTION

According to a first mode, the first variable inputted to the production device and the second variables including at least one state variable related to the target product and an intermediate produced in a process of obtaining the target product from the raw material are acquired by the variable acquisition section. The estimation section estimates the state of the production device on the basis of the first variable and the second variables acquired by the variable acquisition section. Therefore, the state can be monitored in real time.

According to a second mode, in the first mode, the estimation section includes a life estimation section which estimates a life of a part which constitutes the production device. It is possible to prepare parts to be replaced (e.g., consumable parts) in accordance with the estimated lives of the parts.

According to a third mode, in the second mode, the second variables include at least one particular variable selected from the load factor of a compressor for compressing a gas, the concentration of the intermediate, the production amount of the intermediate, the concentration of the target product, the production amount of the target product, and the amount of water produced in a process of obtaining the target product. The life estimation section estimates the life of the part on the basis of the first variable and the particular variable. Thus, it is possible to improve the accuracy of estimating the life of the part.

According to a fourth mode, in the third mode, the life estimation section includes a pre-learned neural network which has undergone machine learning for relating the particular variable to the life of the part. It is possible to optimize the timing of replacement of the part, thereby increasing the operation rate of the production device.

According to a fifth mode, in any of the second to fourth modes, an order section orders a replacement part for the part that reaches the end of its life on the basis of an output of the life estimation section. Therefore, it is possible to easily manage parts to be replaced such as consumable parts.

According to a sixth mode, in any of the first to fourth modes, a changing section changes the first variable inputted to the production device on the basis of an output of the estimation section. Therefore, it is possible to increase the operation rate of the production device while extending the lives of the parts.

### BRIEF DESCRIPTION OF DRAWINGS

[FIG. 1] FIG. 1 is a block diagram of a production device in one embodiment.
[FIG. 2] FIG. 2 is a perspective view of a mechanical apparatus.
[FIG. 3] FIG. 3 is a block diagram of the mechanical apparatus.
[FIG. 4] FIG. 4 is a block diagram of a monitoring device.
[FIG. 5] FIG. 5 is a block diagram of the production device.
[FIG. 6] FIG. 6 is a table showing an example of information stored in a storage device.
[FIG. 7] FIG. 7 is a diagram schematically showing a model of a neuron.
[FIG. 8] FIG. 8 is a diagram schematically showing a neural network configured by combining neurons.

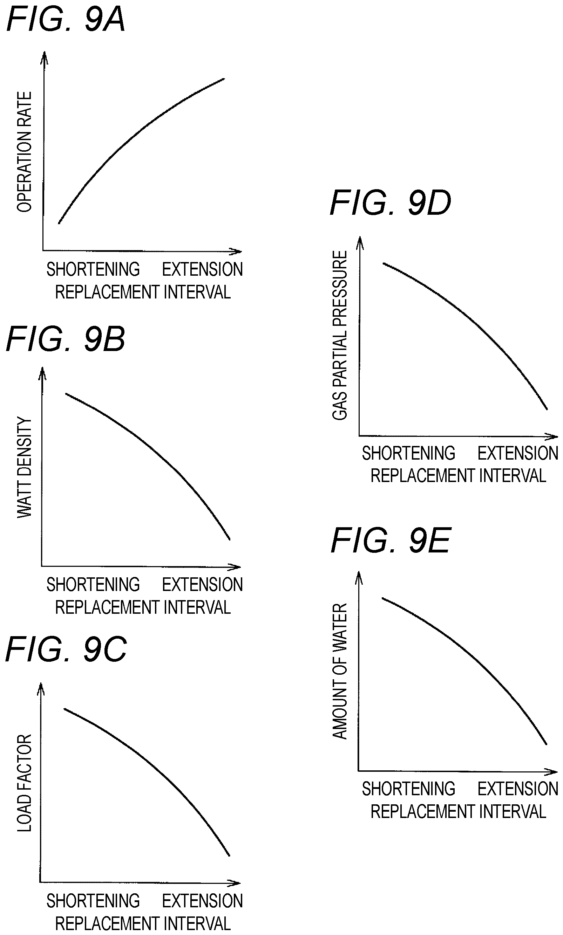

[FIG. 9] FIG. 9A is a graph schematically showing a correlation between part replacement interval and the operation rate of the mechanical apparatus, FIG. 9B is a graph schematically showing a correlation between replacement interval of a heater and the watt density of the heater, FIG. 9C is a graph schematically showing a correlation between part replacement interval and the load factor of a compressor, FIG. 9D is a graph schematically showing a correlation between part replacement interval and the gas partial pressure of a target product, and FIG. 9E is a graph schematically showing a correlation between part replacement interval and the amount of water produced in the process of obtaining the target product.
[FIG. 10] FIGS. 10A and 10B are schematic graphs showing conversion, to reward, of the relation between the operation rate of the mechanism apparatus and the gas partial pressure of the target product.
[FIG. 11] FIG. 11 is a flowchart showing one example of operation of the monitoring device.
[FIG. 12] FIG. 12 is a table showing one example of an action value table.

### DESCRIPTION OF EMBODIMENT

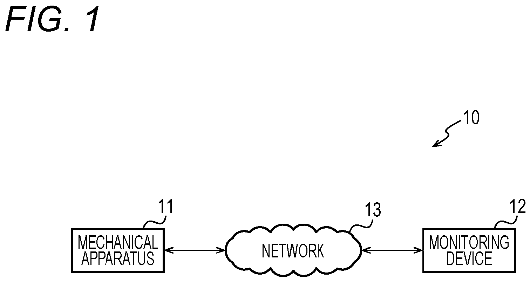

A preferred embodiment of the present invention will now be described with reference to the attached drawings. FIG. 1 is a block diagram of a mechanical apparatus 11 in one embodiment. The mechanical apparatus 11 includes a mechanical apparatus 11 which obtains a target product from a raw material through a chemical reaction or purification, and a monitoring device 12 which monitors the mechanical apparatus 11. The mechanical apparatus 11 and the monitoring device 12 are connected to each other via a network 13. Examples of the network 13 include mobile phone networks, wireless LANs, wired LANs, fixed-line telephone networks, Internet, intranets, and Ethernet (registered trademark).

Although the monitoring device 12 is independent from the mechanical apparatus 11 in FIG. 1, a portion or the entirety of the monitoring device 12 may be contained in the mechanical apparatus 11. Although the monitoring device 12 is illustrated as a single device in FIG. 1, the monitoring device 12 may be composed of a plurality of devices which operate in cooperation with one another. In this case as well, a portion or the entirety of the monitoring device 12 may be contained in the mechanical apparatus 11. The mechanical apparatus 11 may be remotely monitored by using a monitoring device 12 disposed away from the mechanical apparatus 11.

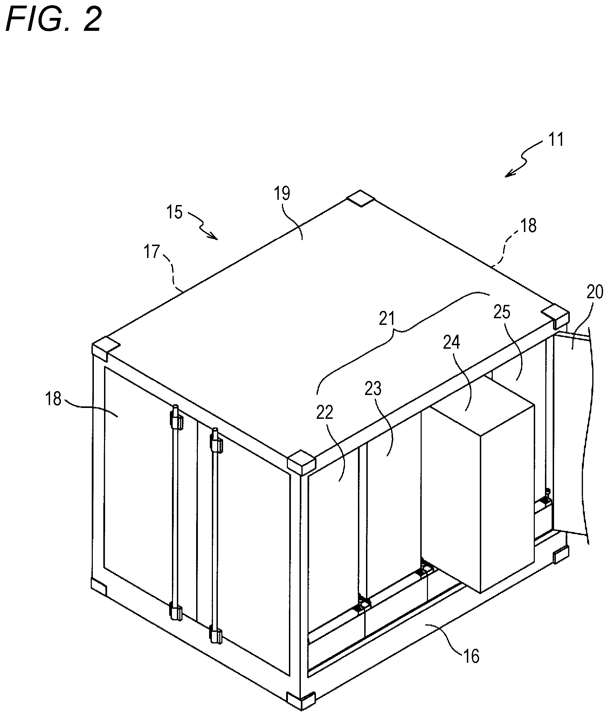

FIG. 2 is a perspective view of the mechanical apparatus 11. The mechanical apparatus 11 includes a transport container 15 and a plurality of functional units 21 accommodated in a transport container 15. In the present embodiment, four functional units 21 are disposed in the transport container 15.

The transport container 15 is a large rectangular parallelepipedic container which is formed mainly of steel material and is used for freight transport. Since the functional units 21 are accommodated in the transport container 15, it is possible to assemble the mechanical apparatus 11 at a plant, transport the assembled mechanical apparatus 11 to a site as it is, and install it at the site. Therefore, it becomes possible to eliminate the necessity of a large scale construction for installing the mechanical apparatus 11 at the site. Also, the capacity of a facility can be increased easily by stacking the mechanical apparatuses 11 or arraying them laterally.

The transport container 15 includes a base 16 having a rectangular shape as viewed from above, a rear wall 17 provided along a longer side of the base 16, two side walls 18 provided along shorter sides of the base 16, a roof 19 which connects the rear wall 17 and the side walls 18, a front door (double doors) 20 provided along a longer side of the base 16 located opposite the rear wall 17. A portion of the front door 20 is not shown. The mechanical apparatus 11 is usually operated in a state in which the front door 20 is closed. In the present embodiment, each of the rear wall 17 and the side walls 18 is composed of double doors. However, as a matter of course, each of the rear wall 17 and the side walls 18 can be formed of a plate which cannot be opened and closed.

The functional unit 21 is a unit of an apparatus which plays a specific role. The mechanical apparatus 11 produces a target product from a raw material through combined use of a plurality of functional units 21. The plurality of functional units 21 are arrayed laterally in a row from one side wall 18 of the transport container 15 toward the other side wall 18 of the transport container 15. The functional units 21 are elongated cuboids whose sizes are approximately equal to one another. In the present embodiment, the functional units 21 include a power supply unit 22, an electrolysis unit 23, a recovery unit 24, and a production unit 25.

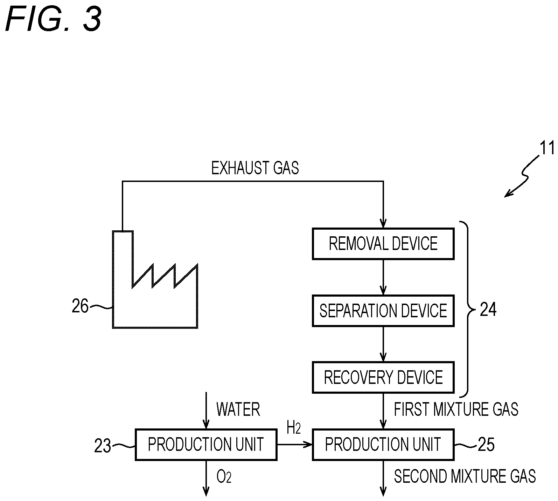

FIG. 3 is a block diagram of the mechanical apparatus 11. The mechanical apparatus 11 which recovers carbon dioxide contained in exhaust gas generated by an exhaust gas source 26 and obtains a target product by recycling the carbon dioxide as a carbon compound will be described below as one example. No particular limitation is imposed on the exhaust gas source 26 so long as the exhaust gas source 26 generates exhaust gas containing carbon dioxide. Examples of the exhaust gas source 26 include an electric power plant, a factory, a waste treatment facility, a natural gas field, and an oil field.

The power supply unit 22 (see FIG. 2) provided in the mechanical apparatus 11 supplies electric power to respective devices. The electrolysis unit 23 includes an electrolysis device for producing hydrogen and oxygen by electrolysis of water. The recovery unit 24 includes a removal device for removing water from the exhaust gas, a separation device for separating nitrogen oxides contained in the exhaust gas, a recovery device for separating carbon dioxide contained in the exhaust gas and condensing the separated carbon dioxide, and a compressor for compressing the gas so as to increase its pressure. The production unit 25 includes a production device for producing a target product by reducing carbon dioxide with hydrogen. Examples of the combustible product produced by the mechanical apparatus 11 include methane, carbon monoxide, methanol, and formaldehyde.

Examples of the method for removing water (water vapor) from the exhaust gas in the removal device of the recovery unit 24 include condensation, physical absorption, and chemical reaction. Examples of common methods for removing nitrogen oxides from the exhaust gas in the separation device are a wet method using caustic soda or the like and a dry method using an NOx removal catalyst and a reducing agent to reduce nitrogen oxides to nitrogen. Removal of water of the exhaust gas by the removal device and removal of nitrogen oxides from the exhaust gas make it possible to secure an efficiency of condensation of carbon dioxide by the recovery device.

A first mixture gas containing carbon dioxide separated and recovered from the exhaust gas in the recovery device is supplied to the production unit 25 (the production device). In the production device, for example, a catalyst is used to reduce the activation energy, thereby proceeding a chemical reaction from carbon dioxide to the target product.

The first mixture gas may contain impurities other than carbon dioxide in an amount of 10 vol% or more of the first mixture gas. It is preferred that the amount of impurities contained in the first mixture gas is small because the purity of the target product contained in a second mixture gas discharged from the production device increases. However, this requires a complex device for separating impurities from the first mixture gas. Accordingly, from the viewpoint of simplification of the mechanical apparatus 11, a certain degree of mixing of impurities is permissible.

Examples of the method for electrolyzing water in the electrolysis unit 23 (the electrolysis device) include electrolysis of alkaline water, electrolysis of water by the mediation of a solid polymer electrolyte, and electrolysis of high-temperature steam by the mediation of a solid oxide electrolysis cell (SOEC). High-temperature steam electrolysis is preferable to alkaline water electrolysis and solid polymer electrolyte water electrolysis because it can produce a large amount of hydrogen with less power. In the case where the electrolysis device performs high-temperature steam electrolysis using SOEC and heat of chemical reaction generated by the production device is used to generate steam for the high-temperature steam electrolysis, the energy efficiency of the mechanical apparatus 11 increases. Therefore, high-temperature steam electrolysis is preferred.

Although the second mixture gas discharged from the production device is allowed to contain, in addition to the target product, hydrogen and the components of the first mixture gas, the amount of gases which are contained in the second mixture gas and are other than the target product is preferably equal to or less than 45 vol% of the amount of the second mixture gas.

It is preferred that the second mixture gas produced by the mechanical apparatus 11 is utilized by a facility in the site which contains the exhaust gas source 26, because the cost associated with transport of the gas can be reduced. Since the size of the mechanical apparatus 11 can be reduced by simplifying it, a space necessary for installing the mechanical apparatus 11 can be reduced. Since the mechanical apparatus 11 can be individually installed for each exhaust gas source 26, carbon dioxide discharged from each exhaust gas source 26 can be individually recycled for the exhaust gas source 26 as a carbon source. The mechanical apparatus 11 can reduce emission of carbon dioxide, while producing a combustible product of the minimum quality required for the use of the combustible product by facilities in the site containing the exhaust gas source 26, rather than producing a combustible product for sale.

Main chemical reactions in the mechanical apparatus 11 of the present embodiment are as follows.
Hydrogen production: 2H₂O → 2H₂ + O₂
Methane production (methanation): CO₂ + 4H₂ → CH₄ + 2H₂O

In hydrogen production, water is a raw material, and hydrogen is a target product. In methane production, carbon dioxide and hydrogen are raw materials, and methane is a target product.

Also, the mechanical apparatus 11 may be configured in such a manner that several reactions shown as examples below occur.
Synthesis gas production by steam reforming: CH₄ + H₂O → CO + 3H₂
Synthesis gas production by dry reforming: CH₄ + CO₂ → 2CO + 2H₂
Synthesis gas production by partial oxidation of methane: 2CH₄ + O₂ → 2CO + 4H₂ Methanol synthesis: CO + 2H₂ → CH₃OH
Fischer-Tropsch (FT) synthesis : CO + 2H₂ → -(CH₂)- + H₂O (-(CH₂)- represents a linear-chain hydrocarbon)
Dimethyl ether (DME) synthesis: 2CO + 4H₂ → CH₃OCH₃ + H₂O

In methanol synthesis through hydrocarbon production and synthesis gas production, carbon dioxide and hydrogen are raw materials, methane and synthesis gas (CO + H₂) are intermediates, and methanol is a target product. In FT synthesis through hydrocarbon production and synthesis gas production, carbon dioxide and hydrogen are raw materials, methane and synthesis gas are intermediate products, and linear-chain hydrocarbon is a target products. In DME synthesis through hydrocarbon production and synthesis gas production, carbon dioxide and hydrogen are raw materials, methane and synthesis gas are intermediate products, and DME is a target product. The mechanical apparatus 11 can use, as the raw materials for the production of methane and synthesis gas, carbon dioxide purified from exhaust gas and oxygen purified from air.

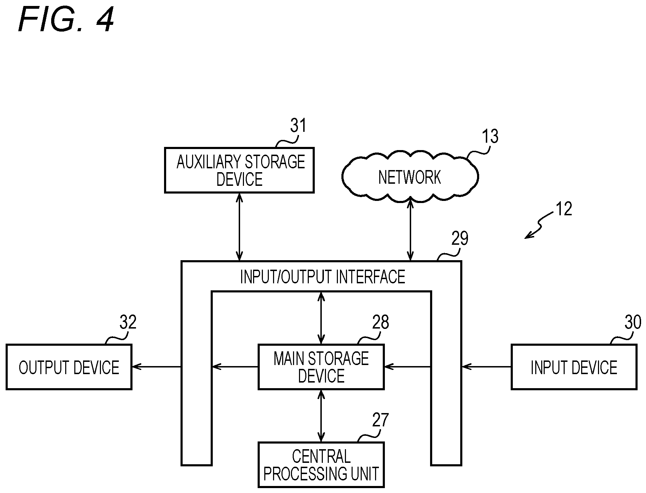

FIG. 4 is a block diagram showing the hardware of the monitoring device 12. The monitoring device 12 includes a central processing unit 27, a main storage device 28, an input/output interface 29, an input device 30, an auxiliary storage device 31, and an output device 32. These are connected by a data bus and a control bus.

The central processing unit 27 can perform operation for instructions and data stored in the main storage device 28 and store the results of the operation in the main storage device 28. The central processing unit 27 can control the input device 30, the auxiliary storage device 31, and the output device 32 via the input/output interface 29.

The main storage device 28 can store instructions and data which are received from the input device 30, the auxiliary storage device 31, and the network 13 via the input/output interface 29 and the results of operation by the central processing unit 27. Examples of the main storage device 28 include random-access memory (RAM), read-only memory (ROM), and flash memory.

The auxiliary storage device 31 is a storage device whose capacity is larger than that of the main storage device 28. The auxiliary storage device 31 can store instructions and data constituting a particular application and an operating system. Various pieces of information regarding parts which constitute the mechanical apparatus 11 are stored in the auxiliary storage device 31. Examples of the auxiliary storage device 31 include a magnetic disk device, and an optical disk device.

The input device 30 is a device for providing data, pieces of information, instructions to the central processing unit 27. Examples of the input device 30 include a touch panel, a button, a keyboard, and a mouse. The output apparatus 32 is a device for physically showing data to the outside. Examples of the output device 32 include a display, a touch panel, and a printer.

The central processing unit 27 can write the instructions and data, which are stored in the auxiliary storage device 31 and constitute the particular application, into the main storage device 28, and perform execution of the instructions, operation, etc. The central processing unit 27 controls the output device 32 via the input/output interface 29 and exchanges (transmits and receives) various pieces of information between the central processing unit 27 and the mechanical apparatus 11 via the input/output interface 29 and the network 13.

The mechanical apparatus 11 (see FIG. 1) includes a controller (not shown) composed of hardware similar to that of the monitoring device 12. The controller exchanges (transmits and receives) various pieces of information between the controller and the monitoring device 12. In addition, the controller coordinates the control instructions of the functional units 21 and controls the functional units 21. Since the controller of the mechanical apparatus 11 is composed of hardware similar to that of the monitoring device 12, description of the controller will be omitted.

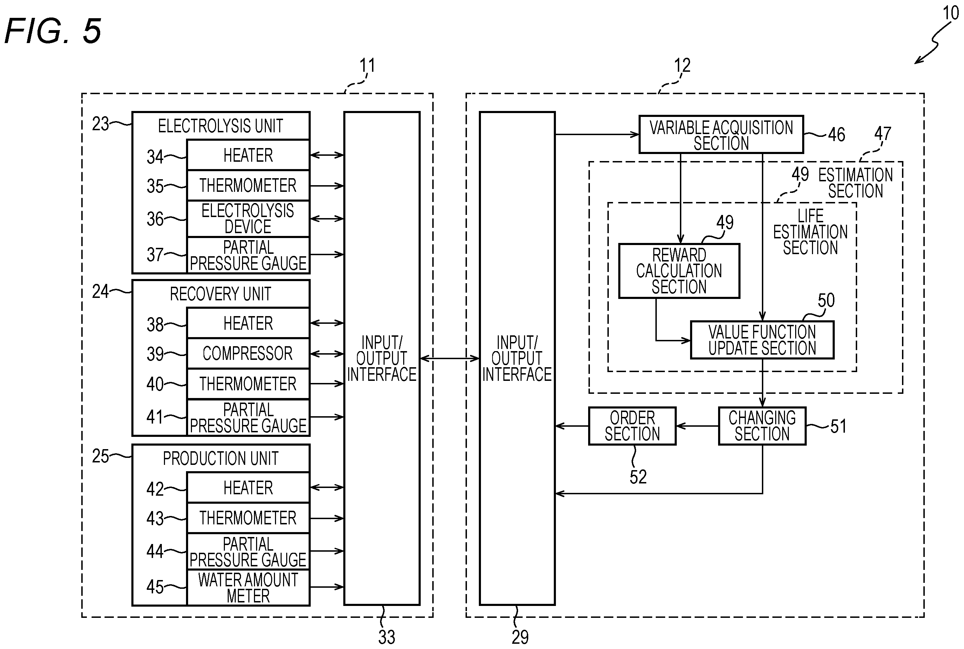

FIG. 5 is a block diagram of the production device 10. The electrolysis unit 23, the recovery unit 24, and the production unit 25 of the mechanical apparatus 11 are connected to the monitoring device 12 via the input/output interface 33. In order to simplify the description, in the present embodiment, the following case will be described as one example. The electrolysis unit 23 performs electrolysis of high-temperature steam by the mediation of an SOEC. The recovery unit 24 captures carbon dioxide in exhaust gas into an absorbing solution by a so-called chemical absorbing method, then heats the absorbing solution to separate the carbon dioxide, cools and compresses the carbon dioxide, and recovers the carbon dioxide. The production unit 25 produces water in the process of obtaining methane by methanation using a catalyst.

The electrolysis unit 23 includes a heater 34 for heating water, thereby producing water vapor, a thermometer 35 for detecting the temperature of the heater 34, an electrolysis device 36 for electrolyzing water vapor, and a partial pressure gauge 37 for detecting the gas partial pressures of hydrogen and oxygen obtained by steam electrolysis.

The recovery unit 24 includes a heater 38 for heating the absorbing solution, a compressor 39 for compressing the carbon dioxide separated from the absorbing solution, a thermometer 40 for detecting the temperature of the absorbing solution, and a partial pressure gauge 41 for detecting the gas partial pressure of the carbon dioxide.

The production unit 25 includes a heater 42 for heating a reaction vessel containing a catalyst, a thermometer 43 for detecting the temperature of the reaction vessel, a partial pressure gauge 44 for detecting the partial pressure of the produced methane, and water amount meter 45 for detecting the amount of water produced in the process of obtaining methane.

The monitoring device 12 includes a variable acquisition section 46 for acquiring various variables (hereinafter referred to as "first variables") inputted to the mechanical apparatus 11 and various variables outputted from the mechanical apparatus 11 (hereinafter referred to as "second variables"), and an estimation section 47 for estimating the state of mechanical apparatus 11 on the basis of the first and second variables acquired by the variable acquisition section 46. The monitoring device 12 further includes a changing section 51 for changing the first variables inputted to the mechanical apparatus 11 on the basis of the output of the estimation section 47.

Examples of the first variables include the amount of water (raw material) supplied to the electrolysis unit 23, the amount of gas (raw material) supplied to the production unit 25, the electric power supplied to the heaters 34, 38, and 42, the electrolysis device 36, and the compressor 39, which are necessary to achieve a target operation rate of the mechanical apparatus 11. The operation rate of the mechanical apparatus 11 is an index representing the ratio of the amount of a target product (methane in the present embodiment) actually produced to the production capacity of the mechanical apparatus 11. The first variables changed by the changing section 51 are inputted to the controller (not shown) of the mechanical apparatus 11 via the input/output interfaces 29 and 33. The controller controls the functional units 21 of the mechanical apparatus 11 so as to produce the target product.

Examples of the second variables include the power consumptions of the heaters 34, 38, and 42, the electrolysis device 36, and the compressor 39, the temperatures of the object heated by the heaters 34, 38, and 42, which are detected by the thermometers 35, 40, and 43, the amount and pressure of the gas used by the compressor 39, the gas partial pressures (concentrations) detected by the partial pressure gauges 37, 41, and 44, the amount of methane produced, and the amount of water detected by the water amount meter 45. The operation rate of the mechanical apparatus 11 and the load factor of the compressor 39, which are computed by using these variables, are also the second variables. The load factor of the compressor 39 is the ratio of the amount of gas discharged by the compressor 39 to the amount of gas used by the compressor 39.

The monitoring device 12 causes the changing section 51 to change the first variables inputted to the mechanical apparatus 11, thereby feedback-controlling the mechanical apparatus 11. The estimation section 47 of the monitoring device 12 includes a life estimation section 48 for estimating the lives of parts which constitute the mechanical apparatus 11.

For example, as the compressor 39 degrades, its pressurization performance decreases, and the power consumption required to increase the pressure of gas to a certain pressure increases. Thus, the life estimation section 48 can estimate the life of the compressor 39 on the basis of the load factor and power consumption of the compressor 39. Also, as the heaters 34, 38, and 42 deteriorate, their heating performances decrease, and the power consumption required to heat the object to be heated to a certain temperature increases. Thus, the life estimation section 48 can estimate the lives of the heaters 34, 38, and 42 on the basis of the relationship between the temperature of the object to be heated and the power consumptions (e.g., watt densities) of the heaters 34, 38, and 42. Watt density is the power per unit surface area of the heaters 34, 38, and 42.

When the catalyst of the production unit 25 and the absorbing solution of the recovery unit 24 deteriorate, the concentration of the produced gas, the concentration of the purified gas, the amount of methane produced decrease, and the amount of water produced in the process of obtaining methane increases or decreases. The increase or decrease in the amount of water may occur due to distortion or damage of the internal piping of the mechanical apparatus 11. Accordingly, the life estimation section 48 can estimate the lives of the catalyst and the absorbing solution on the basis of the concentration of gas, the amount of water, and the amount of methane produced, and can estimate the life of the piping on the basis of the amount of water.

The life estimation section 48 outputs a piece of information about a part reaching the end of its life to the changing section 51, and the order section 52 orders a part which replaces the part reaching the end of its life, on the basis of the output of the changing section 51. Thus, it is possible to easily manage parts to be replaced, such as consumable parts constituting the production device 10. Notably, the absorbing solution used in the recovery unit 24 of the mechanical apparatus 11 and the catalyst used in the production unit 25 are also contained in the parts constituting the production device 10.

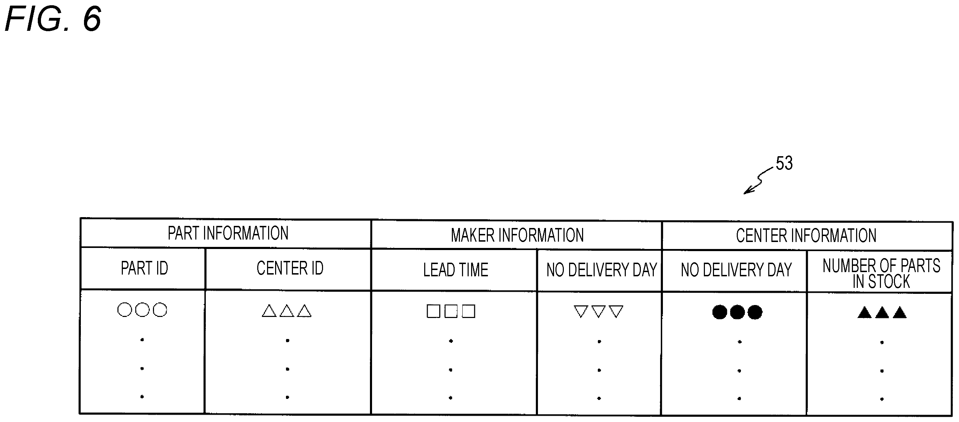

FIG. 6 is a table showing one example of the information (part table 53) stored in the auxiliary storage device 31. The part table 53 may be stored in the main storage device 28. The order section 52 can refer to the part table 53 and automatically place an order with a center for the parts recorded in the part table 53 via the network 13 (see FIG. 1). The center is a retailer or wholesaler which sells parts and with which orders for parts are placed.

The part table 53 includes a piece of information for identifying each part (part ID), a piece of information for identifying each center from which the part is ordered (center ID), a piece of information about the center (hereinafter referred to as the "center information"), and a piece of information about a maker which manufacture the part (hereinafter referred to as the "maker information"). The maker information is a piece of information about a manufacturer which manufactures the part specified by the part ID, and contains pieces of information about lead time (the time needed for the center to receive the part after having placed an order for the part with that maker) and days on which the maker does not deliver the part (hereinafter referred to as "no delivery days"). The center information contains a piece of information about the no delivery days of the center and a piece of information about the number of parts in stock, which is the quantity of parts in the warehouse of the center. The maker and the center can input the maker information and the center information of the part table 53.

Referring back to FIG. 5, the description of the order section 52 will be continued. The order section 52 places an order for the part with the center, in consideration of the center's stock and the lead time recorded in the part table 53 (see FIG. 6), so that the part is shipped to a location where the mechanical apparatus 11 is installed by the time the part is replaced. The order section 52 may cause the output device 32 (see FIG. 4) to provide an output (e.g., generation of a sound or display of an image) for prompting an operator to place an order. The operator who has confirmed the output can place an order for the part with the center.

The life estimation section 48 includes a reward calculation section 49 for calculating a reward on the basis of the first variables and the second variables acquired by the variable acquisition section 46, and a value function update section 50 for updating a function that determines the optimal value of the interval of part replacement due to deterioration, on the basis of the reward calculated by the reward calculation section 49.

It is possible to reduce the power consumption of the mechanical apparatus 11 or improve the quality of the target product produced by the mechanical apparatus 11 by replacing a deteriorated part, among the parts which constitute the mechanical apparatus 11, with a new part. In some cases, it is possible to increase the production amount of the target product. Therefore, replacing a deteriorated part with a new one reduces the production cost of the target product, as compared to production of the target product performed by using the mechanical apparatus 11 containing the deteriorated part.

On the other hand, since the replacement of parts is performed with the mechanical apparatus 11 stopped, the higher the frequency of part replacement (shorter the replacement interval), the longer the stop time of the mechanical apparatus 11, and the lower the operation rate of the mechanical apparatus 11. In addition, the higher the frequency of part replacement, the higher the cost of replacing parts, and the higher the production cost. It is necessary to select the optimal interval for replacing parts by considering these factors.

In view of the above, the reward calculation section 49 gives a small reward on the basis of, for example, shortening of the part replacement interval, an increase in the difference between the actual temperature of the object to be heated and its predicted value, an increase in the difference between the actual power consumption of the heaters and its predicted value, an increase in the difference between the actual density of the gas and its predicted value, an increase in the difference between the actual load factor of the compressor and its predicted value, an increase in the difference between the actual amount of water and its predicted value, an increase in the difference between the actual production amount of the target product and its predicted value, and a decrease in the operation rate of the mechanical apparatus 11.

On the contrary, the reward calculation section 49 gives a large reward on the basis of extension of the part replacement interval, a decrease in the difference between the actual temperature of the object to be heated and its predicted value, a decrease in the difference between the actual power consumption of the heaters and its predicted value, a decrease in the difference between the actual density of the gas and its predicted value, a decrease in the difference between the actual load factor of the compressor and its predicted value, a decrease in the difference between the actual amount of water and its predicted value, a decrease in the difference between the actual production amount of the target product and its predicted value, and an increase in the operation rate of the mechanical apparatus 11.

The value function update section 50 updates a part replacement action value table (value function) on the basis of the reward calculated by the reward calculation section 49. The value function is stored in the main storage device 28 or the auxiliary storage device 31.

The estimation section 47 may have a function of extracting useful rules, knowledge expressions, judgment criteria, etc. from a set of data inputted to the monitoring device 12 by analysis, and outputting judgment results, and perform knowledge learning (machine learning). There are various methods of machine learning, but they can be broadly divided into supervised learning, unsupervised learning, and reinforcement learning. Furthermore, there is a method called deep learning that learns extraction of features themselves in order to realize these methods. Machine learning can be achieved by applying a GPGPU (General Purpose computing with Graphic Processing Unit), a large-scale PC cluster, or the like. Reinforcement learning will be described below as an example, but the machine learning is not limited to this.

Reinforcement learning sets up a problem as follows. The production device 10 detects the state of the environment and determines an action (replacement of parts). The environment changes according to some rules, and its own action can also change the environment. Every time the production device 10 acts, a reward signal is fed back to the production device 10. What we want to maximize is the sum of rewards over the future. Learning begins with no knowledge of the consequences of an action, or an incomplete knowledge of the consequences. In other words, the production device 10 can obtain the result as data only after actually taking an action. Namely, the production device 10 searches for the best action through trial and error. As if imitating human behavior, the production device 10 may start learning from a good starting point by using, as an initial state, the state in which pre-learning (supervised learning, inverse reinforcement learning, etc.) has been performed.

In reinforcement learning, not only judgment and classification but also actions are learned, whereby the optimal action is learned in consideration of the interaction of the action on the environment; that is, learn how to maximize the reward obtained in the future. This means that it is possible to acquire actions that affect the future; for example, replacement of parts which constitute the mechanical apparatus 11, which action determines the state quantity of the production unit 25 (the concentration of the target product, the amount of water, etc.). A neural network can be used as an approximation algorithm for the value function in reinforcement learning.

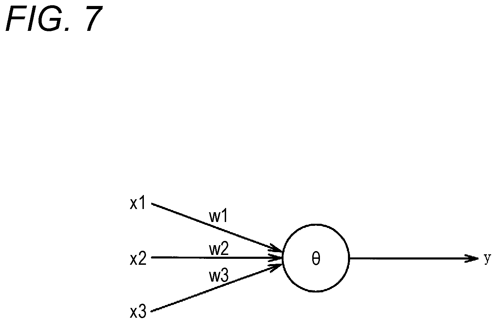

FIG. 7 is a diagram schematically showing the model of a neuron. A neuron outputs an output (result) y for a plurality of inputs x. Each input x (x1, x2, x3) is multiplied by a weight w (w1, w2, w3) corresponding to the input x. The inputs x, the result y, and the weights w are all vectors.

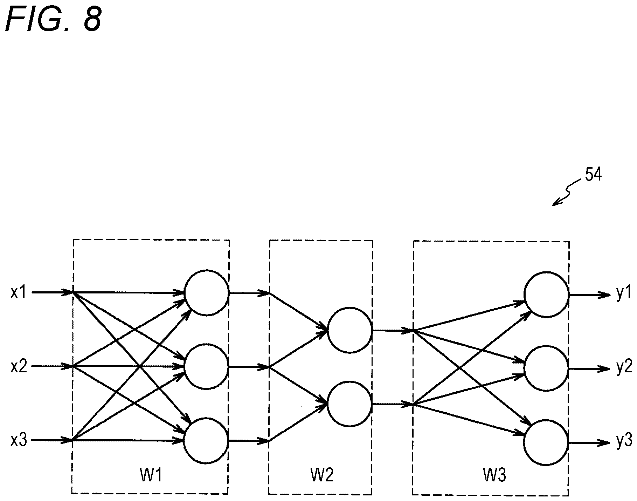

FIG. 8 is a diagram schematically showing a neural network 54 configured by combining neurons. The neural network 54 includes an arithmetic unit which imitates the model of a neuron, a memory, etc. In the present embodiment, the neural network 54 has three layers. However, the number of layers is not limited to three. It is naturally possible to increase the number of layers to 3 or more.

The neural network 54 multiplies weights W1, W2, and W3 corresponding to a plurality of inputs x (x1, x2, and x3 in the present embodiment) in order, and outputs the results y (y1, y2, and y3 in the present embodiment). The weights W1, W2, and W3 can be learned by the error backpropagation method.

Operation of the neural network 54 includes a learning mode and a prediction mode. For example, in the learning mode, the weights W are learned by using a training data set, and action judgment is made in the prediction mode using its parameters. In the predictive mode, a variety of tasks such as detection, classification, and inference are possible. The neural network 54 can learn in real time the data obtained by actually operating the production device 10 in the prediction mode, and reflect them on the next action (online learning). In addition, learning may be performed collectively by using a data group collected in advance, and then the prediction mode may be executed by using the parameters (batch learning). Alternatively, it is possible to execute learning that is in between online learning and batch learning; i.e., to execute the prediction mode and execute the learning mode every time a certain amount of data is accumulated.

FIG. 9A to FIG. 9E are graphs used for describing one example of operation of the estimation section 47. In each drawing, the horizontal axis shows part replacement interval, and the vertical axis shows the parameter of each part. The horizontal axis means that the larger the value (the farther to the right), the longer the part replacement interval. The vertical axis means that the smaller the value (the lower the coordinate position in the vertical axis), the lower the operation rate, or the greater the degree of deterioration of the part.

FIG. 9A is a graph schematically showing a correlation between the part replacement interval and the operation rate of the mechanical apparatus 11. When the part replacement interval is extended, since the operation time of the mechanical apparatus 11 becomes longer, the operation rate of the mechanical apparatus 11 increases. Meanwhile, when the part replacement interval is shortened, since the operation time of the mechanical apparatus 11 becomes shorter, the operation rate of the mechanical apparatus 11 decreases.

FIG. 9B is a graph schematically showing a correlation between heater replacement interval (the replacement interval of each of the heaters 34, 38, and 42) and the watt density of each heater. When the replacement interval of each of the heaters 34, 38, 42 is extended, each of the heaters 34, 38, and 42 deteriorates with time. Meanwhile, when the replacement interval of each of the heaters 34, 38, 42 is shortened, a state in which the power consumption of each of the heaters 34, 38, and 42 is approximately equal to its predicted value is maintained.

FIG. 9C is a graph schematically showing a correlation between the replacement interval of parts of the compressor 39 and the load factor of the compressor 39. When the replacement interval of parts of the compressor 39 is extended, the compressor 39 deteriorates with time. Meanwhile, the replacement interval of parts of the compressor 39 is shortened, a state in which the load factor of the compressor 39 is approximately equal to its predicted value is maintained.

FIG. 9D is a graph schematically showing a correlation between the part replacement interval and the gas partial pressure (concentration) of the target product. When the part replacement interval is extended, the gas partial pressure of the target product decreases with time (the difference between the actual gas partial pressure of the target product and its predicted value increases gradually). Meanwhile, the part replacement interval is shortened, a state in which the gas partial pressure of the target product is approximately equal to its predicted value is maintained.

FIG. 9E is a graph schematically showing a correlation between the part replacement interval and the amount of water produced in the process of obtaining the target product. When the part replacement interval is extended, the difference between the amount of water produced in the process of obtaining the target product and its predicted value increases. Meanwhile, when the part replacement interval is shortened, a state in which the amount of water is approximately equal to its predicted value is maintained.

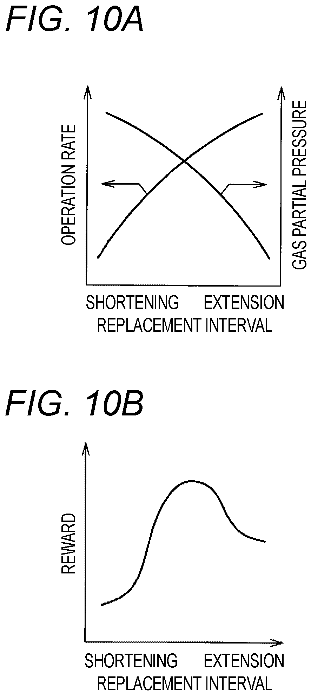

FIG. 10A shows the relation between the operation rate of the mechanical apparatus 11 and the gas partial pressure (concentration) of the target product. The intersection between two curves in FIG. 10A shows a proper part replacement interval obtained by the estimation section 47. FIG. 10B is a schematic graph showing the reward converted from that relation. The estimation section 47 cumulatively adds various rewards, learns to maximize the total of cumulatively added rewards, and finds the optimal replacement intervals of various parts. The point at which a part is replaced is when the life of the part is exhausted.

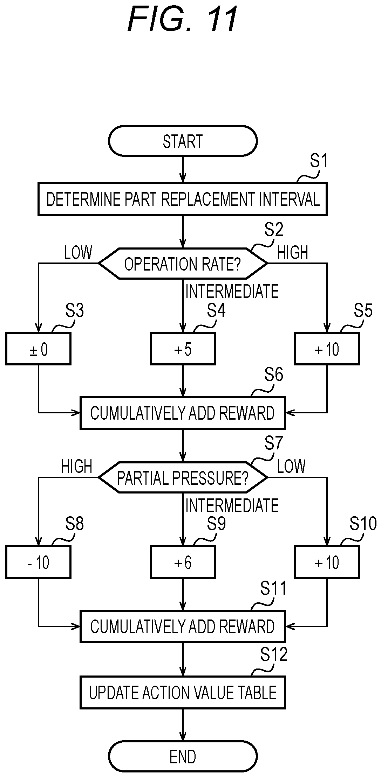

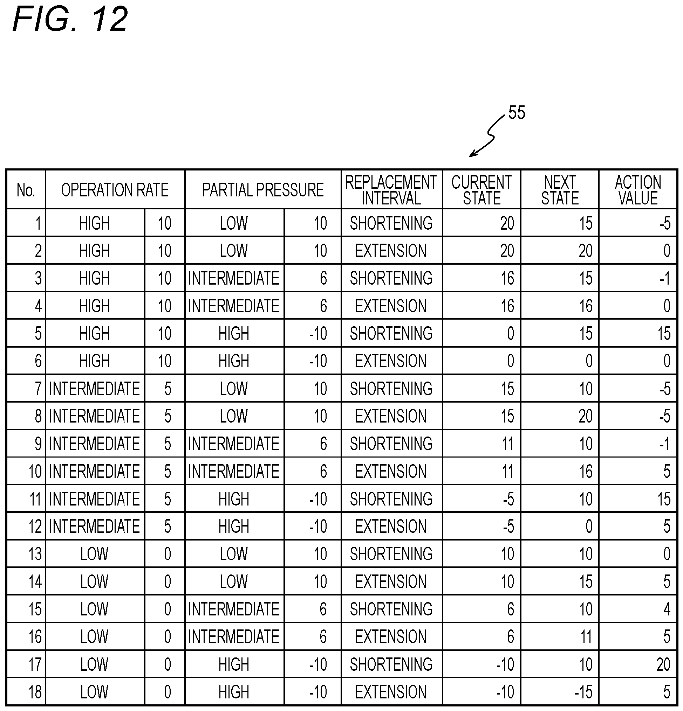

FIG. 11 is a flowchart showing one example of operation of the monitoring device 12. The monitoring device 12 first determines the part replacement interval on the basis of an action value table 55 (see FIG. 12) (S1), judges the operation rate of the mechanical apparatus 11 (S2), and determines a reward (S3 to S5). For example, when the operation rate of the mechanical apparatus 11 is low, the monitoring device 12 outputs no reward (reward is 0) (S3). When the operation rate of the mechanical apparatus 11 is at an intermediate level, the monitoring device 12 outputs a reward of +5 (S4). When the operation rate of mechanical apparatus 11 is high, the monitoring device 12 outputs a reward of +10 (S5). The monitoring device 12 cumulatively adds the outputted reward to a previous reward(s) (S6).

Next, the monitoring device 12 compares the actual gas partial pressure of the target product with its predicted value (S7), and determines a reward (S8 to S10). For example, when the difference between the actual gas partial pressure of the target product and its predicted value is large, the monitoring device 12 outputs a reward of -10 (S8). When the difference between the actual gas partial pressure and its predicted value is at an intermediate level, the monitoring device 12 outputs a reward of -6 (S9). When the difference between the actual gas partial pressure and its predicted value is small, the monitoring device 12 outputs a reward of +10 (S10). The monitoring device 12 cumulatively adds the outputted reward to the previous reward(s) (S11), and updates the action value table 55 on the basis of the total of the rewards (S12).

The processing of S1 to S12 is performed repeatedly while the power supply of the monitoring device 12 is on. The reward values of S3 to S5 and S8 to S10 are examples and can be changed as appropriate. Of course, it is possible to execute the processing of S2 to S6 and the processing of S7 to S11 in parallel.

FIG. 12 is a table showing one example of the action value table 55. Patterns No. 1 to No. 18 are recorded in the action value table 55. Patterns No. 1 to No. 6 are for the case where the operation rate of the mechanical apparatus 11 is high (reward is +10), patterns No. 7 to No. 12 are for the case where the operation rate of the mechanical apparatus 11 is at an intermediate level (reward is +5), and patterns No. 13 to No. 18 are for the case where the operation rate of the mechanical apparatus 11 is low (reward is 0). In patterns No. 1 to No. 18, when shortening of the part replacement interval is chosen, the reward related to the operation rate of the mechanical apparatus 11 is decreased to a value of a lower next rank, and the reward related to the gas partial pressure of the target product is increased to a value of a higher next rank. When extension of the part replacement interval is chosen, the reward related to the operation rate of the mechanical apparatus 11 is increased to a value of a higher next rank, and the reward related to the gas partial pressure of the target product is not changed.

Description is provided by using patterns No. 9 and No. 10 as an example. The patterns No. 9 and No. 10 are for the case where the operation rate of the mechanical apparatus 11 is at an intermediate level (reward is +5) and the difference between the actual gas partial pressure of the target product and its predicted value is at an intermediate level (reward is +6). In both patterns, the total of the rewards in the current state is +11. When shortening of the part replacement interval is chosen at that time, the reward related to the operation rate is decreased to a value of a lower next rank, so that 0 is outputted as a reward, and the reward related to the gas partial pressure of the target product is increased to a value of a higher next rank, so that +10 is outputted as a reward. Therefore, +10 is outputted as the reward of the next state. The value (+10 - 11 = -1) obtained by subtracting the reward of the current state from the reward of the next state is the value of the action of choosing shortening of the part replacement interval.

Meanwhile, when extension of the part replacement interval is chosen, the reward related to the operation rate is increased to a value of a higher next rank, so that +10 is outputted as a reward, and the reward related to the gas partial pressure of the target product does not change, so that +6 is outputted as a reward. Therefore, +16 is outputted as the reward of the next state. The value (+16 - 11 = +5) obtained by subtracting the reward of the current state from the reward of the next state is the value of the action of choosing extension of the part replacement interval.

Since comparison between the value (-1) of the action of choosing shortening of the part replacement interval and the value (+5) of the action of choosing extension of the part replacement interval reveals that the value of the action of choosing extension of the part replacement interval is higher, the production device 10 choses extension of the part replacement interval. Since the production device 10 can obtain proper timings of replacement of the parts which constitutes the mechanical apparatus 11 in the above-described manner and can maintain the mechanical apparatus 11 properly, the operation rate of the mechanical apparatus 11 can be optimized. However, these are mere examples, and, as to selection of actions and setting of rewards, various modifications and changes are possible. The action value table 55 is also one example, and various modifications and changes are possible.

In addition, according to the production device 10, since the estimation section 47 estimates the state of the mechanical apparatus 11 on the basis of the first variables and the second variables acquired by the variable acquisition section 46, the state of the mechanical apparatus 11 can be monitored in real time. Furthermore, since the estimation section 47 includes the life estimation section 48 which estimates the lives of the parts which constitute the mechanical apparatus 11, parts to be replaced, such as consumable parts, can be prepared in accordance with the estimated lives of the parts. Since the parts can be replaced properly, it is possible to prevent occurrence of a problem that, due to deterioration of parts, the operation rate of the mechanical apparatus 11 abnormally decreases, or the mechanical apparatus 11 becomes inoperable.

The second variables acquired by the variable acquisition section 46 of the production device 10 include at least one particular variable selected from the load factor of the compressor 39, the concentration (gas partial pressure) of the target product, the production amount of the target product, and the amount of water produced in the process of obtaining the target product. Since the life estimation section 48 estimates the lives of parts on the basis of the first variables and the particular variable, it is possible to improve the accuracy of estimating the lives of the parts. Notably, in the case where the production device 10 produces the target product through an intermediate such as methane or synthetic gas, at least one of the concentration of the intermediate and the production amount of the intermediate may be contained in the particular variable.

Since the life estimation section 48 includes the pre-learned neural network 54 which has undergone machine learning for relating the particular variable to the lives of the parts, the timings of replacement of the parts can be optimized, whereby the operation rate of the production device 10 can be increased. In addition, since the changing section 51 changes the first variables inputted to the mechanical apparatus 11 on the basis of the output of the estimation section 47, it is possible to increase the operation rate of the mechanical apparatus 11 while extending the lives of the parts which constitute the mechanical apparatus 11.

Although the present invention has been described on the basis of an embodiment, it can be easily surmised that the present invention is not limited to the above-described embodiment, and various improvements and modifications can be made without departing from the purpose of the present invention.

In the embodiment, there has been described the mechanical apparatus 11 which includes the functional units 21 composed of the electrolysis unit 23, the recovery unit 24, and the production unit 25, in addition to the power supply unit 22. However, the present invention is not limited thereto. It is of course possible to dispose various functional units 21 in accordance with the role which the mechanical apparatus 11 plays, thereby enabling the mechanical apparatus 11 to play other roles. Examples of mechanical apparatuses 11 which play other roles include a module for producing hydrogen and oxygen by using water as a raw material, a module for purifying carbon dioxide from exhaust gas, a module for producing an intermediate, such as synthetic gas, methanol, or ethanol, from carbon dioxide and hydrogen, and producing fuels (e.g., diesel oil and gasoline), BTX, DME, butadiene, chemical products, etc. from the intermediate.

In the embodiment, there has been described the case where the state updated by the action of changing the part replacement interval (the operation rate of the mechanical apparatus 11 and the difference between the actual gas partial pressure of the target product and its predicted value) and a reward are fed back to the production device 10. However, the present invention is not limited thereto. It is of course possible to give a reward(s) to one or more differences in place of or in addition to the operation rate of the mechanical apparatus 11 and the difference between the actual gas partial pressure of the target product and its predicted value, and feed the reward(s) back to the production device 10. The one or more differences are selected from the difference between the actual temperature of the object to be heated and its predicted value, the difference between the actual power consumption of the heaters and its predicted value, the difference between the actual density (gas partial pressure) of the intermediate and its predicted value, the difference between the actual production amount of the target product and its predicted value, the difference between the actual production amount of the intermediate and its predicted value, the difference between the actual load factor of the compressor and its predicted value, and the difference between the amount of water produced as a result of production of the target product and its predicted value, etc.

In the embodiment, there has been described the case where the estimation section 47 uses an algorithm (machine learning) that automatically improves by learning from experience. However, the present invention is not limited thereto. It is of course possible to obtain the correlation between one or more factors and changing of the part replacement interval, and control the mechanical apparatus 11 or estimate the lives of the parts which constitute the mechanical apparatus 11 on the basis of the correlation. The one ore more factors are selected from the operation rate of the mechanical apparatus 11, the difference between the actual gas partial pressure of the target product and its predicted value, the difference between the actual production amount of the target product and its predicted value, the difference between the actual gas partial pressure of the intermediate and its predicted value, the difference between the actual production amount of the intermediate and its predicted value, the difference between the actual temperature of the object to be heated and its predicted value, the difference between the actual power consumption of the heaters and its predicted value, the difference between the actual load factor of the compressor and its predicted value, and the difference between the amount of water produced as a result of production of the target product and its predicted value, etc.

In the embodiment, there has been described the case where four functional units 21 are installed in the transport container 15 which allows installation of up to four functional units 21. However, the present invention is not limited thereto. The number of the functional units 21 installed in the transport container 15 is freely set in accordance with the purpose of the mechanical apparatus 11. The transport container 15 may be configured to allow installation of five or more functional units 21 or may have vacancies therein.

The present disclosure can be realized as the following modes.

### [Application example 1]

A production device for obtaining a target product from at least one type of raw material selected from liquid and gas through a chemical reaction or purification, the production device comprising;
a variable acquisition section which acquires a first variable inputted to the production device and second variables including at least one state variable related to the target product and an intermediate produced in a process of obtaining the target product from the raw material; and
an estimation section which estimates the state of the production device on the basis of the first variable and the second variables acquired by the variable acquisition section.

### [Application example 2]

The production device described in application example 1, wherein the estimation section includes a life estimation section which estimates a life of a part which constitutes the production device.

### [Application example 3]

The production device described in application example 2, wherein the second variables include at least one particular variable selected from the load factor of a compressor for compressing the gas, the concentration of the intermediate, the production amount of the intermediate, the concentration of the target product, the production amount of the target product, and the amount of water produced in a process of obtaining the target product, and
the life estimation section estimates the life of the part on the basis of the first variable and the particular variable.

### [Application example 4]

The production device described in application example 3, wherein the life estimation section includes a pre-learned neural network which has undergone machine learning for relating the particular variable to the life of the part.

### [Application example 5]

The production device described in any of application examples 2 to 4, further comprising an order section for ordering a replacement part for the part that reaches the end of its life on the basis of an output of the life estimation section.

### [Application example 6]

The production device described in any of application examples 1 to 5, further comprising a changing section which changes the first variable inputted to the production device on the basis of an output of the estimation section.

### REFERENCE SIGNS LIST

- 10:: production device
- 39:: compressor
- 46:: variable acquisition section
- 47:: estimation section
- 48:: life estimation section
- 51:: changing section
- 52:: order section
- 54:: neural network

## Claims

1. A production device for obtaining a target product from at least one type of raw material selected from liquid and gaseous materials through a chemical reaction or purification, the production device comprising:
a variable acquisition section which acquires a first variable inputted to the production device and second variables including at least one state variable related to the target product and an intermediate produced in a process of obtaining the target product from the raw material; and
an estimation section which estimates the state of the production device on the basis of the first variable and the second variables acquired by the variable acquisition section.

2. A production device according to claim 1, wherein the estimation section includes a life estimation section which estimates a life of a part which constitutes the production device.

3. A production device according to claim 2, wherein the second variables include at least one particular variable selected from the load factor of a compressor for compressing the gas, the concentration of the intermediate, the production amount of the intermediate, the concentration of the target product, the production amount of the target product, and the amount of water produced in a process of obtaining the target product, and
the life estimation section estimates the life of the part on the basis of the first variable and the particular variable.

4. A production device according to claim 3, wherein the life estimation section includes a pre-learned neural network which has undergone machine learning for relating the particular variable to the life of the part.

5. A production device according to any of claims 2 to 4, further comprising an order section for ordering a replacement part for the part that reaches the end of its life on the basis of an output of the life estimation section.

6. A production device according to any of claims 1 to 4, further comprising a changing section which changes the first variable inputted to the production device on the basis of an output of the estimation section.
